# EUROPEAN PATENT SPECIFICATION

(11) **EP 0 600 136 B1**
(45) Date of publication and mention of the grant of the patent: **13.03.2002**
(21) Application number: 92403248.5
(22) Date of filing: 01.12.1992
(51) Int. Cl.: C12N 15/12, C07K 14/00, C12P 21/08, G01N 33/68, C12N 5/10, C12N 1/21

(54) **Intron/Exon structure of the human and mouse Beta 3 Adrenergic receptors genes**
Intron/Exon Struktur der Genen von den menschlichen und Maus Beta 3 adrenergischen Rezeptoren
Structure de introns/exons de gènes codants pour les récepteurs bêta 3 adrénergiques souris et humain

(43) Date of publication of application: 08.06.1994
(73) Proprietor: CENTRE NATIONAL DE LA RECHERCHE SCIENTIFIQUE, 75007 Paris (FR)
(72) Inventor: Emorine, Laurent, F-75013 Paris (FR); Strosberg, A. Donny, F-75015 Paris (FR); Nahmias-Kaminski, Clara, F-75003 Paris (FR)
(74) Representative: Desaix, Anne

(56) References cited:
- WO-A-90/08775
- WO-A-92/12246
- KAPLAN, J.C.; DELPECH, M. 'Biologie Moléculaire et Médecine' 1989 , FLAMMARION MEDECINE-SCIENCES , PARIS, FRANCE

## Description

### BACKGROUND OF THE INVENTION

The present invention relates to the entire gene encoding β3-adrenergic receptor polypeptides of mouse and human origin, which polypeptides provide a procedure for studying the effects of various chemical agents on the β3-adrenergic receptor and coupled adenylate cyclase and hormone-sensitive lipases. More particularly, the present invention also relates to antibodies, vectors, nucleotide probes, cell hosts transformed by genes encoding polypeptides, having β3-adrenergic receptor activity.

In the past, two main classes of adrenergic receptors have been identified as the a adrenergic receptors and the β adrenergic receptors. These adrenergic receptors produce various responses to effector organs such as the eye, heart, arteriols, veins, lungs, stomach, intestine, gallbladder, kidney, skin, spleen, liver and pancrease to mention a few. These specific receptors have been defined by the effects of particular synthetic agonists which stimulate the receptors biological function and antagonists which block the adrenergic receptors biological function.

Within the two classes of adrenergic receptors, four subtypes, α1, α2, β1 and β2, of these receptors for catecholamines have been identified. See, Cotecchia et al., P.N.A.S., 85, pgs. 7159-7163 (1988); Kobilka et al., Science 238, pgs. 650-656 (1987); Frielle et al., P.N.A.S., 84, pgs. 7920-7924 (1987); and Emorine et al., P.N.A.S., 84, pgs. 6995-6999 (1987). Drugs that selectively block or stimulate one of these receptor subtypes are used extensively in clinical medicine. Despite the efficacy of these drugs, many produce side effects in individuals, due to their interaction with other receptor subtypes. For identification of the various drugs which act on the receptor subtypes, see Goodman and Gilman's The Pharmacological Basis of Therapeutics, eigth edition, MacMillan Publishing Co., Inc. (1990).

The analysis of the genes of these receptors indicate that all of the subtypes of the receptors belong to the family of integral membrane receptors exhibiting striking homologies, in particular these homologies are present in the 7 transmembrane regions. These receptors are coupled to regulatory proteins termed G proteins which are capable of binding molecules of guanosine triphosphate (GTP).

The G proteins have the capacity to intervene structurally and functionally between receptors and enzymes catalyzing the production of intracellular mediators such as adenylate cyclase, guanylate cyclase, the phospholipases and the kinases, or between receptors and ion channels. Thus, the G proteins have the capacity to regulate the flux of ions such as calcium, potassium, sodium and hydrogen ions.

The above-mentioned subtypes of receptors are known in the art as the "R₇G family" as described by Emorine et al. Proc. NATO Adv. Res. Workshop (1988). The R₇G family comprises acetylcholine muscarinic receptors, serotonin receptors, the receptors for neuropeptides, substance K, angiotensin II and the visual receptors for the opsins, as described by Dixon et al Annual Reports in Medicinal Chemistry, pgs. 221-233 Ed. Seamon, K.B., Food and Drug Administration, Bethesda, Md. (1988) and Emorine et al., supra.

Recently, a third subtype of the β-adrenergic receptor termed β3-adrenergic receptor, has been identified and characterized in humans and in rodents, which polypeptide does not have a receptor activity similar to that of the β1- or β2-adrenergic receptors. This "new" β3-receptor in humans has been identified and sequenced as described in French application No.8900918 filed January 25, 1989 and PCT/FR90/00054, resulting in U.S. application No.07/721,571, filed January 25, 1990, incorporated herein by reference, as containing 402 amino acids, which is capable of activating adenylate cyclase in the presence of an agonist, which activity increases in the order of agonists of salbutamol, BRL 28410, BRL 37344 and (1)-isoproterenol. Antibodies directed to the polypeptide of the β3-adrenergic receptor were also disclosed. The β3-receptor was identified as differing from the β1-adrenergic receptor and the β2-adrenergic receptor by a pharmacological comparison of the activation of adenylate cyclase in the presence of agonists and the reaction towards different antagonists.

Similarly, the β3-adrenergic receptor of the mouse has been identified and cloned as described in French application No. 9100320 filed January 14, 1991 and PCT/FR92/00023 filed January 15, 1992, also incorporated herein by reference. See also, Nahmias et al., Embo J., 10, pgs. 3721-3727 (1991). The mouse β3-adrenergic receptor encodes a polypeptide of 388 amino acid residues, including the features characteristic of β-adrenergic receptors, such as the conserved amino acids identified as crucial for catecholamine binding in the β2-adrenergic receptor. See, Strader et al.. FASEB J. 3, pgs. 1825-1832 (1989).

A pharmacological comparison of the mouse with the human counter part of the β3-adrenergic receptors indicates that these receptors have similar reactivity. For example, mouse β3-adrenergic receptor can be activated by CGP 12177A, oxprenolol and pindolol while displaying a low stereoselectivity and characteristic potency order for full agonists, which is similar to the human.

However, differences do exist between the mouse β3-adrenergic receptor and the human β3-adrenergic receptor, which are probably due to the structural differences observed between these two receptors in the transmembrane domains which are involved in ligand binding wherein 12 substitutions do occur.

Thermogenesis and lipolysis in brown and white adipose tissues are under the control of this β3-adrenergic receptor subtype as described by Arch et al., Proc. Nutr. Sci., 48, pgs. 215-223 (1989); Arch et al., In Obesity, John Wiley & Sons Ltd., London (1991); and Zaagsma et al., Trends Pharmacol. Sci., 11, pgs. 3-7 (1990). Besides adipose tissues, the expression of the β3-adrenergic receptor has also been reported in various other tissues such as tissues of the digestive tract, from oesophagus to colon and in the gallbladder. See, for example, Bond et al., Br. J. Pharmacol., 95, pgs. 723-734 (1988); Coleman et al., British Journal of Pharmacology Proc. Supl., 90, 40 (1987); Bianchetti et al., Br. J. Pharmacol., 100, pgs. 831-839 (1990); Granneman et al., J. Pharmacol. Exp. Ther., 256, pgs. 421-425 (1991); and Krief et al., J. Clin. Invest., (1993) (in press). This β3-adrenergic receptor subtype has been suggested to participate in the control by catecholamines of body energy balance from intestine assimilation to storage and mobilization in adipose tissue.

Factors such as temperature, feeding or fasting and stress influence the body's hormonal status, thus inducing tissue specific adaptive modifications of energy balance which may in part result from regulation of cellular β3-adrenergic receptor sensitivity. It has been further shown that β-adrenergic agonists, glucocorticoids and several other agents can modulate β3-adrenergic receptor density, responsiveness and mRNA levels. The molecular determinants responsible for the regulation of cellular β3-adrenergic receptor may possibly be found either on the receptor itself or in its gene or mRNA. For example, post-translational modifications of the receptor in the third intracytoplasmic loop or in the carboxy-terminal tail may modulate β-adrenergic receptor coupling to adenylate cyclase. Factors which act on β-adrenergic receptor gene transcription rate or on mRNA stability may also modulate cellular adrenergic responsiveness by modifying β-adrenergic receptor expression levels.

The β3-adrenergic receptors have been sequenced and identified, as well as compared pharmacologically with other known receptors in mouse and humans. A comparison of the β3-adrenergic receptor amino acid sequences predicted from the nucleotide sequences of the human and mouse genomic genes revealed differences in the carboxy-terminal regions of the receptors. Although these differences could be attributed to evolutionary species-related variations, it was recently discovered by the present inventors that the entire coding sequence for the previously reported β3-adrenergic receptor in mouse and human was not complete. The genes encoding β1- and β2- comprise one exon and it was thought that the β3-adrenergic receptor gene, when initially identified, also comprised only one exon. However, it was recently and unexpectedly discovered that contrary to β1- and β2-adrenergic receptor genes, the human and mouse β3-adrenergic receptor genes comprise several exons. The identification of the introns and exons in mouse and human β3-adrenergic receptors and thus the entire gene itself, permits a full characterization of this receptor which will aid in more sensitive genetically engineered products such as nucleotide probes, polyclonal and monoclonal antibodies and the like for drug testing and diagnostic purposes, and permits the understanding of β3-adrenergic receptor regulated expression in various tissues.

### SUMMARY OF THE PRESENT INVENTION

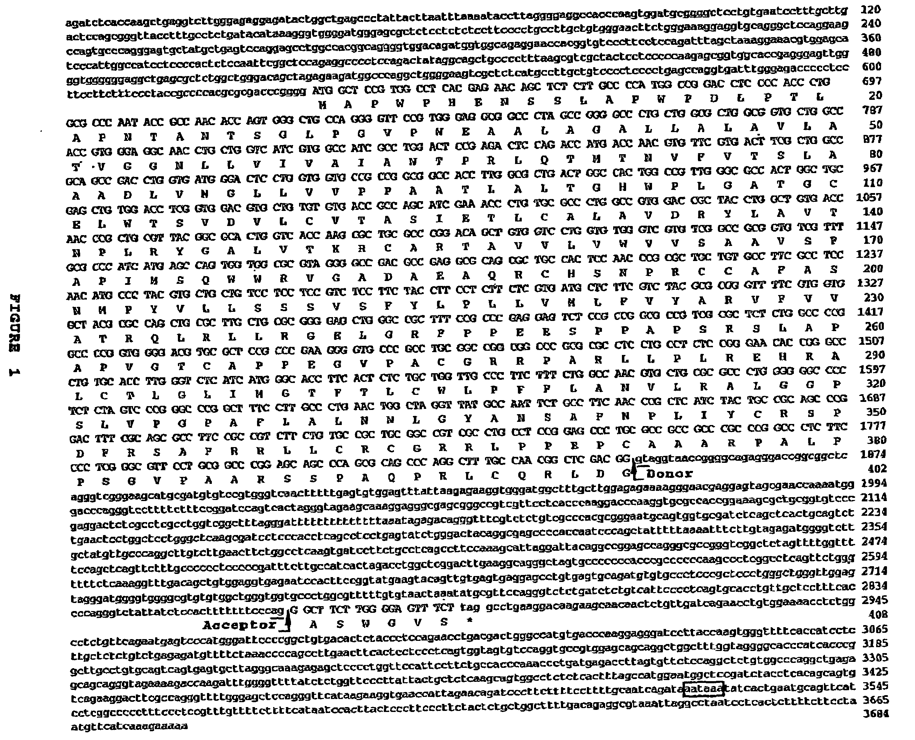

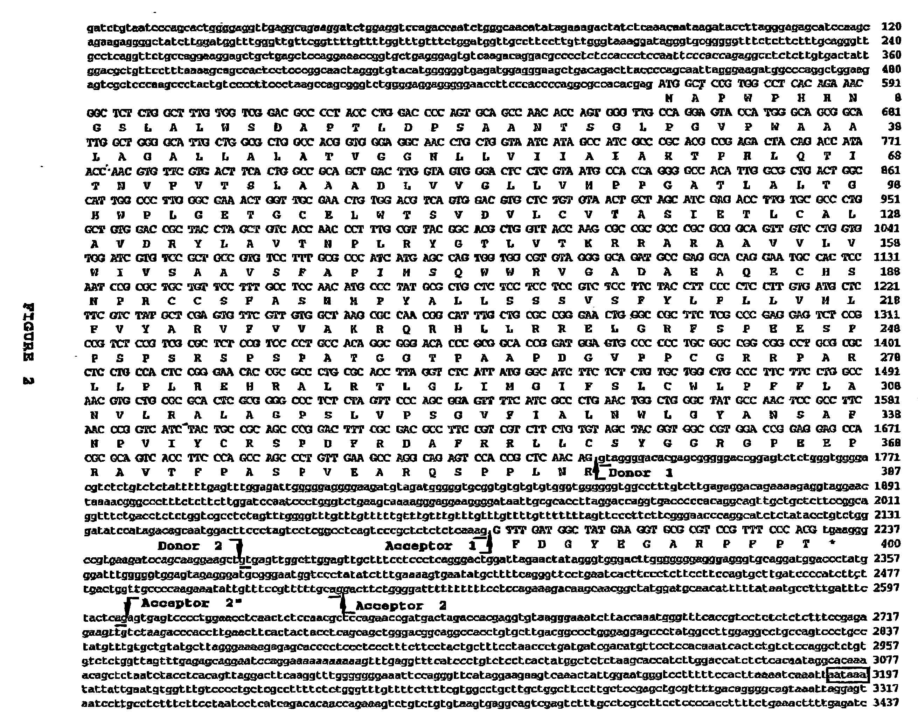

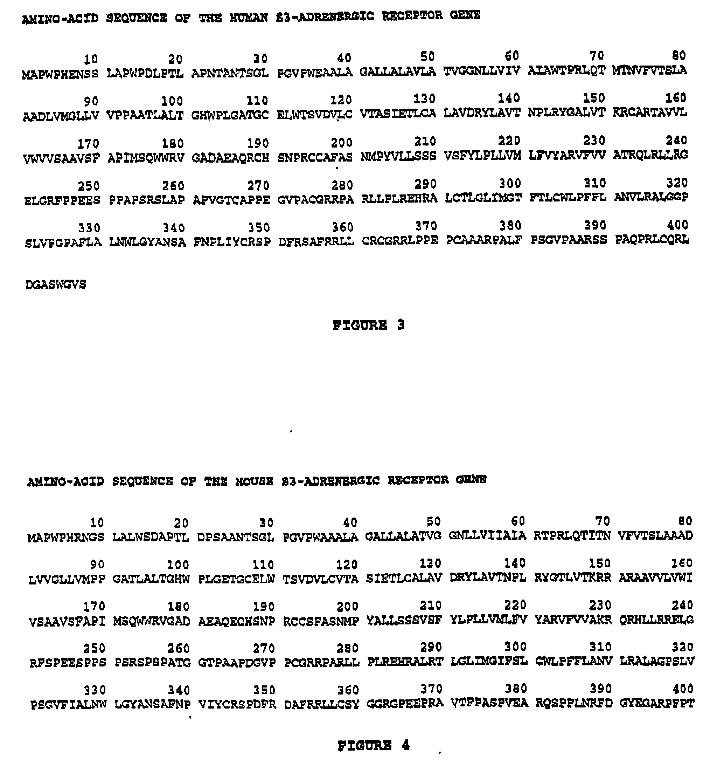

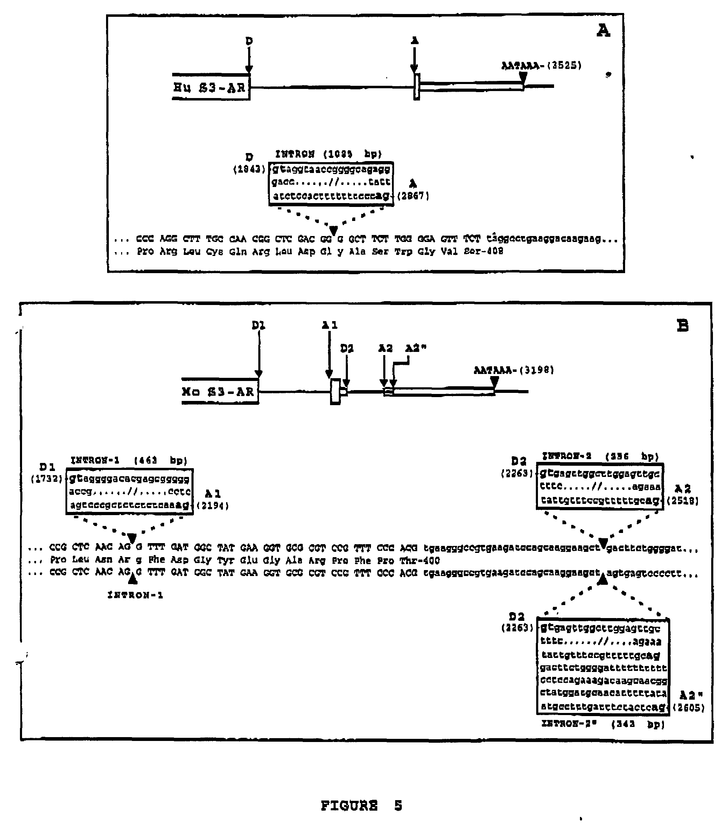

Thus, it is an object of the present invention to provide an isolated and purified nucleic acid sequence comprising the nucleotide sequence of Figure 1 and Figure 2.

Yet another object of the present invention is to provide polypeptides having β3-adrenergic receptor activity in mammals which polypeptides encode the entire human and mouse β3-adrenergic receptor genes.

A specific object of the present invention is to provide the entire amino acid and nucleotide sequences, as well as the intron/exon organization for the human and mouse β3-adrenergic receptor which molecule characterizations are useful in not only detecting the β3-adrenergic receptor in different species, but also testing new drugs for regulating β3-adrenergic receptor activities.

Yet another object of the present invention is to provide nucleotide probes capable of hybridizing to the genes encoding the polypeptides having β3-adrenergic activity in mammals for detecting this receptor in different mammalian species and for measuring the variations in the levels of expression of the β3-receptor in mammalian cells.

Another object of the present invention is to provide antibodies, polyclonals and monoclonals which recognize the entire sequence of the β3-adrenergic receptor in mammals, which antibodies do not recognize the β1-adrenergic receptor or β2-adrenergic receptor for detecting and diagnostic purposes.

A further object of the present invention is to provide recombinant vectors for the cloning and expression of β3-adrenergic receptor proteins in mammals.

Another object of the present invention is to provide a cell host which comprises the elements of regulation making possible the expression of the nucleotide sequence encoding the polypeptides of β3-adrenergic receptors in mammals.

### BRIEF DESCRIPTION OF THE DRAWINGS

Fig. 1 depicts the nucleotide sequence, amino acid translation and intron/exon organization of the human β3-adrenergic receptor gene.

Fig. 2 depicts the nucleotide sequence, amino acid translation and intron/exon organization of the mouse β3-adrenergic receptor gene.

Fig. 3 is the complete amino acid sequence for the human β3-adrenergic receptor.

Fig. 4 is the complete amino acid sequence for the mouse β3-adrenergic receptor.

Fig. 5 is a schematic representation of human and mouse β-adrenergic receptor mRNA splicing.

### DESCRIPTION OF THE PREFERRED EMBODIMENTS

In particular, the present invention provides various genetically engineered products such as nucleotide probes, monoclonal and polyclonal antibodies for testing a variety of chemical agents including drugs, ligands and the like which may influence the regulation of β3-adrenergic receptors. Moreover, in this testing method, it is of importance to have the entire sequence of the β3-receptor gene, since elements in the promoter region, in the intron/exon region and in the 3' untranslated region may influence the expression of β3. By testing drugs that may increase and/or decrease the expression levels of the β3-adrenergic receptor, various diseases such as obesity, diabetes and hyperlipidemia and the like, may be controlled.

Accordingly, the present invention also provides a method for in vitro diagnosis of energy-balance related diseases which diseases can be diagnosed by abnormalities in β3-adrenergic receptor activity.

The present invention also provides vectors containing genes coding for polypeptides having β3-adrenergic receptor activity in mammals, as well as cell hosts transformed by genes coding for the above-mentioned polypeptides.

By mammals is meant any class of higher vertebrates comprising man and all other animals that nourish their young with milk secreted by mammary glands and have skin, usually more or less covered by hair, including monkeys, mice, rat, humans and the like.

Any mammalian polypeptide displaying β3-adrenergic receptor activity is a part of the present invention, but preferable polypeptides encoding the amino acid sequences set forth in Figures 3 and 4.

Variations of the polypeptides is also encompassed by the present invention provided that these variant polypeptides do not lose β3-adrenergic receptor activity. Any technique known in the art may be used to provide these variant polypeptides including nucleotide-mediated mutagenesis, oligonucleotide-mediated "loop out" mutagenesis, linker-inserted mutagenesis and the like. These methods are well-known and described, for example by Sambrook et al. in Molecular Cloning A Laboratory Manual, second edition, Cold Spring Harbour Laboratory Press (1989).

Besides polypeptides, the present invention also encompasses any nucleotide sequence of β3-adrenergic receptors in mammals. A preferred embodiment of these nucleotide sequences are encompassed in Figures 1 and 2. Variants of the nucleotide sequence are also encompassed in the present invention including mutations and point substitutions using the above-described mutagenesis methods, provided that these variations do not significantly alter β3-adrenergic receptor activity.

These nucleotides can be prepared by any synthesis method known in the art. Examples of these methods include, by are not limited to the automated β-cyanoethyl phosphoramidite method described in Bioorganic Chemistry, 4, pgs. 274-325 (1986) which method is suitable for the preparation of a nucleotide sequence containing a maximum of 200 nucleotides and those described in P.N.A.S., 80, pgs. 7461-7465 (1983) for nucleotides longer than 200.

Besides synthesis of nucleotides, the present invention also relates to the purification of nucleotides from genomic DNA or cellular RNA from any mammalian tissue expressing the β3-adrenergic receptor including adipose, muscular, hepatic, intestinal, gallbladder tissues and the like. mRNA can then be isolated and cDNA synthesized therefrom by the methods described in Sambrook et al. Molecular Cloning, supra.

The nucleotide sequences or fragments thereof can be cloned and/or expressed in a plasmid, cosmid or phage type vector. Thus, the present invention also includes recombinant vectors which which can be use to clone or express in a variety of host microorganisms the β3-adrenergic receptor. Various vectors include, but are not limited to pBr322, pUC18/pUC19, pUC119, p5P64/p5P65, pGEM-3/pGEM-4, pGEM-3Z, πAN13, Bluescript M13, λgt10, λ2001, λDASH, λFIX, C2RB, pWE15 and the like.

A preferred vector includes a HindII-PstI fragment of genomic DNA derived from mouse having about 5.5 kilobases and containing the totality of the gene coding for the β3-adrenergic receptor in mouse including the promoter region and Exon 1, Exon 2 and Exon 3, which fragment was inserted into a HindII-PstI site of pUC18. The plasmid was transformed in E. coli(JM101). This vector, (030 B315-I) was deposited under the No. I-1272 on December 1, 1992 with the C.N.C.M., Collection Nationale de Cultures de Microorganismes, 25 rue du Docteur Roux, Paris France.

A second preferred vector includes a BglII fragment of genomic DNA derived from humans having about 4.5 kilobases and containing the totality of the gene coding for the β3-adrenergic receptor in humans including the promoter region and Exon 1 and Exon 2, which fragment was inserted into a BamHI site of pUC18. The plasmid was then transformed in E. coli (JM101). This vector, (119 B315-III) was deposited under the No. I-1273 on December 1, 1992 with the C.N.C.M., Collection Nationale de Cultures de Microorganismes, 25 rue du Docteur Roux, Paris France.

For expression the vector contains in at least one of its sites not essential for replication, elements necessary to promote the expression of the polypeptide of the present invention in a cell host. For example, a promoter recognized by the RNA polymerase of the cell host may be used. Any promoter that accomplishes expression can be used, but it is preferable to use an inducible promoter and if necessary, a signal and an anchoring sequences. For expression in eukaryotic cells, the regulatory elements may include the endogenous promoter for the adrenergic receptors or viral promoters such as those of SV40 virus or Rous Sarcoma virus, while for bacterial expression, the regulatory elements may include the lactose operon, the tryptophan operon and the like.

Cell hosts transformed by any of the above-described vectors are also encompassed by the present invention. Any cell host capable of expressing any mammalian β3-adrenergic receptor polypeptide or fragments thereof is encompassed by the present invention. These cell hosts include any eukaryotic or prokaryotic cells including bacteria such as E. coli, yeast, CHO cells and the like. It is of interest to note that when the entire intron/exon sequence of the mouse or human β3-adrenergic receptor is expressed in a variety of eukaryotic cell hosts including CHO and L-cells, splicing of the gene takes place correctly.

The recombinant vectors and cell hosts transformed with these vectors are useful tools to generate with ease and facility various nucleotide probes.

These nucleotide probes can also be prepared from the nucleotide sequences, as discussed above, via synthesis and purification techniques and then labelled with a detectable marker. Any detectable marker can be used to label the specific nucleotide sequence of interest, such as ¹⁴C, ³²P, enzyme markers and the like. The labelling conditions for the sequences are well known in the art and for reference such techniques described by Sambrook et al. Molecular Cloning, supra may be used.

More particularly, it is advantageous to use a full length probe having the nucleotide sequence as defined in Figures 1 and 2 to probe a genomic or cDNA library of different mammalian species to obtain the related β3-adrenergic receptor of interest. A fragment of the nucleotide probe can also be generated.

A nucleotide probe is also useful in measuring the variations in the levels of expression of the β3-adrenergic receptor in mammalian cells, since altered levels of expression may result in specific abnormalities in various mammalian tissues. More specifically, the nucleotide probes may be produced such that they react with specific sequences of the β3-adrenergic receptor gene. Thus, even if this receptor is present in the specific species of mammals being tested, the ligand binding sites or other functional regions in the sequence may contain an abnormality that can be ascertained by the use of such probes.

The nucleotide probes which are suitable for use in the present invention include DNA probes, RNA probes, synthetic oligonucleotide probes, thioester probes and the like. Tandem probes may also be used. The tandem probes may be constructed by covalently joining two or more type-specific β3-adrenergic receptor nucleotide sequences within a single vector. A mixture of the above-mentioned probes is also contemplated for detection purposes.

The conditions required for hybridization and washing of the oligonucleotide probes for detecting the presence of the β3-adrenergic receptor may be different depending on the type of probe utilized. Empirically determined formulae available in the literature allow for the estimation of the oligonucleotide dissociation temperature. Hybridization of oligonucleotides also depend on several factors, which include the length of the probe and the GC content. The hybridization conditions, thus will be adjusted according to the probes used and are well within a person skilled in the art's knowledge.

The labelled hybrids are then detected depending on the type of labelled probe used. For example, an enzymatically labelled probe will require enzymatic detection, while if a radioactive label is used, gamma or beta counters or autoradiography may be used.

The present invention also relates to antibodies directed against the polypeptides of the β3-adrenergic receptor in mammals. These antibodies are specific for the β3-adrenergic receptor and not the β1- nor β2-adrenergic receptors. These antibodies may be produced by injecting the β3-adrenergic receptor protein or polypeptide thereof into an animal. The antibodies generated by the immune response may be recovered and purified by methods known in the art. Besides their use for detection and in vitro diagnostic purposes, the antibodies of the present invention may also be used to purify related receptors in semi-purified form by their attachment to a solid support such as gel beads.

Monoclonal antibodies may be produced by the known method described by Kohler and Milstein, Nature, 256, page 495 (1975). These antibodies are useful to diagnose the amount and the presence of the β3-adrenergic receptor in a variety of mammalian species. Thus, the quantity and presence of this receptor may be important to diagnose diseases related to energy abnormalities in a variety of mammalian tissues.

Although direct detection of the β3-adrenergic receptor polypeptide is possible by the use of genetically engineered products as described above, these products are not limited in use to only detecting the presence of β3-receptors in various species. Testing of different drugs for the treatment of obesity, fatty diabetes, hyperlipidemias and the like are within the scope of the present invention by use of the aforementioned nucleotide probes, β3-adrenergic receptors and anti-receptor antibodies.

A procedure for studying the affinity of a β3-adrenergic receptor polypeptide for one or more chemical agents such as drugs, ligands and the like is also encompassed by the present invention.

To study the effects of various chemical agents, a cultured transformed host cell having a sequence encoding the entire β3-adrenergic receptor gene under conditions allowing the expression of such gene is performed. The expression product, which may be exposed at the surface of the transformed cell host or present in the cytoplasm of the host cell, is then utilized to test a variety of ligands, drugs and chemical agents by placing these agents in contact with the transformed cell host. An affinity reaction between the transformed cell host and the specific agent is then detected.

The transformed host cells containing the polypeptide sequence of the β3-adrenergic receptor can also be used to test intracellular responses to various chemical agents.

More specifically, it is known in the art that a variety of hormones, both peptidic and non-peptidic, exert their cellular effects through interaction with cell surface receptors coupled to guanine nucleotide binding proteins or the G-proteins. The G-protein coupled receptors mediate a variety of intracellular responses, wherein the initial specificity is usually determined at the level of the cell surface receptor. The diversity of the cellular responses results from the differential coupling of the receptor to various G-proteins, each of which stimulates a distinct intracellular effector system. The G-protein linked receptor can bind a variety of agonists which stimulates the G protein coupled to the receptor. The activation of the stimulatory G protein leads in turn to adenylate cyclase stimulation and thus an increase in cAMP levels in cells. The β3-adrenergic receptor has the ability to couple G protein and thus the effects of various agonists to activate the stimulatory G protein, raising cAMP levels in the cell can also be tested using the transformed cell hosts of the present invention.

It is of extreme importance that the entire gene sequence be utilized to conduct the above-identified studies, since it is known that the additional sequences described in the present invention effect the regulation of these receptors and especially influence the receptor's expression. Thus, for instance, to test specific ligands and drugs which may aid in an agent to reduce obesity (since the β3-adrenergic receptor is present in low quantities in obese patients) it may be necessary to increase the amount of β3-receptor present. The RNA expression levels of the β3-adrenergic receptor may be regulated by additional sequences described and characterized in the present invention such as the sequences present in the promoter region, the intron/exon region and the 3' untranslated region. Thus, an agent that can be found to increase the transcription of β3-receptors may lead to treatment for obesity and diabetes.

The cloning and initial sequencing of the human and mouse β3-adrenergic receptor genomic genes have been previously described. Nahmias et al., THE EMBO JOURNAL vol. 16, No. 12 pgs. 3721-3727 (1991) and Emorine et al., Science vol. 245, pgs. 1118-1121 (1989). Both of these references are incorporated herein by reference. To obtain more insight into the mechanisms at the basis of the regulated expression of the β3-adrenergic receptor in both human and mouse, further study was undertaken. By comparing the β3-adrenergic receptor gene and the PCR-amplified cDNA sequences, the presence of introns which interupted the coding and the 3'-untranslated regions of the human and mouse β3-adrenergic receptor gene was discovered.

Nucleotide sequencing was performed on a 2.5 kb SphI-BglII fragment spanning the entire 3'-untranslated regions of the human gene and a 2.2 kb XhoI-BglII fragment spanning the entire 3' untranslated region of the mouse β3-genomic genes after insertion of these fragments in both orientations into M13 cloning vectors. Individual subclones were then sequenced by the standard chain termination method. The primers used were oligonucleotides synthesized step by step during the course of the sequence determination.

A preferred embodiment of the present invention is the identification of two exons in humans and three exons in mice, which contain the entire β3-adrenergic receptor gene sequence. In humans, a large 1.4 kb exon encodes the first 402 amino acid residues, while the second exon of 700 base pairs encodes the sequence for the six carboxy-terminal residues of β3-adrenergic receptor, as well as the entire untranslated 3' region of the β3-adrenergic receptor mRNA.

In mice, there are three exons. The first exon encodes 388 amino acid residues of the β3-adrenergic receptor; a second exon of 68 base pairs encodes for the twelve carboxy-terminal residues of the β3-adrenergic receptor and the third exon of about 600 to 700 base pairs with two acceptor splice sites codes for the 3'-untranslated region of β3-adrenergic receptor mRNA. In mouse adipose tissues, alternate splicing at the two splice sites found in the third exon generates two β3-adrenergic transcripts. The shorter transcript is the major transcript found in white adipose tissue, while the longer transcript is slightly predominant in brown adipose tissue.

Thus, the amino acid sequence for human β3-adrenergic receptor is 6 amino acids longer then previously thought, while the mouse sequence is 12 amino acids longer than previously reported. The nucleotide and amino acid sequences as well as the mRNA splicing are depicted in Figures 1-5 for the human and mouse β3-adrenergic receptor.

The localization of the mRNA transcription start sites was undertaken by S₁ nuclease mapping and the mRNA transcription start sites for both mouse and human β3-adrenergic receptor mRNA were then localized in a region between 150-190 nucleotides upstream from the ATG translation start cocon. In mouse, a second mRNA initiation region exists further upstream. Further upstream from the mRNA cap sites, glucocorticoid response elements were found in the immediate vicinity of recognition sequences for transcription factor AP-1. The inhibitory effects of glucocorticoids on β3-adrenergic receptor mRNA levels may result from interactions of glucocorticoid receptor with c-fos ad c-jun products which constitutes transcription factor AP-1.

Thus, the entire genes including the promoter region, the intron/exon region and the 3'-untranslated region, coding for β3-adrenergic receptors were obtained, as well as the mRNA transcription start sites. This finding is of utmost importance not only for detection and diagnostic purposes, but also to obtain drugs useful in the regulation of β3 expression in tissue. Thus, the utilization of alternate promoters and/or splice sites allows tissue specific regulation of β3-adrenergic expression.

### EXAMPLE 1

### Isolation and sequencing of the murine β3AR gene

All the methods used for recombinant DNA procedures are from Ausubel et al. Science 245, pgs 1118-1121 (1987). A 1.3 kb NcoI-BamHI DNA fragment encompassing the entire coding region of the human β3AR gene (Emorine et al. Current Protocols in Molecular Biology, Greene publishing Associates and Wiley InterScience, New York (1989)) was used as a probe to screen a mouse NIH 3T3 genomic library in the lambda FIX™II vector (Stratagene). The nitrocellulose replica filters of the library were prehybridized for 16 h at 42°C in a buffer containing 8 mM Tris-HCl (pH 7.5), 40% formamide, 4xSSC, 5xDenhardt's, 0.2% SDS, 50 mM sodium phosphate and 100 µg/ml heat-denatured salmon sperm DNA. Hybridization was performed for 16 h at 42°C in the same buffer containing the probe (2x10⁶ c.p.m./ml)³²P-labelled by the random priming method to a specific activity of 10⁹ c.p.m./µg. Final washes were at 45°C in 0.1xSSC and 0.05% SDS.

A 2kb BglII-BamHI restriction fragment hybridizing to the probe was subcloned in both orientation into M13mp18. Nested deletions were created with the exonuclease III and sequencing was performed by the dideoxy chain termination method using [α-³⁵S]dATP (800 Ci/mmol, Amersham) and Thermophilus aquaticus (Tag) polymerase (Taquence kit, USB). Sequence analysis were carried out using the CIT12 (University Paris V, Paris, France) computer software facilities.

### EXAMPLE 2

### DNA probe and Hybridization Analysis

A 310 bp DNA fragment ("A43") corresponding to the N-terminus of the mouse β3-adrenergic receptor was produced by polymerase chain reaction using a template of 5 ng of the lambda clone containing the mouse β3-adrenergic receptor gene. Thirty cycles of amplification at 93°C for 1.5 minute; 55°C for 2 minutes; and 72°C for 2 minutes were performed using 2.5 U Tag polymerase (Cetus) in 100 µl of buffer containing 10 mM Tris-HCl (pH 8.4), 3 mM MgCl₂, 0.05% TWEEN 20, 0.05% NP40, 10% dimethylsuplhoxide, 5% formamide, 125 µM of each deoxynucleotide triphosphate and 125 pM of each primer. Sense (5'-GCTCCGTGGCCTCACGAGAA-3') and antisense (5'-CCCAACGGCCAGTGGCCAGTCAGCG-3') primers corresponding to amino acids 2-8 and 98-106, respectively of the human β3-adrenergic receptor sequence. The amplified 310 bp DNA fragment was purified by electrophoresis through an acrylamide gel. ³²P-labelled by random priming to a specific activity of 10⁹ c.p.m./µg, and used as a probe in Southern and Northern blot experiments.

For Southern blot hybridization analysis, single restriction enzyme digests of genomic DNA (10 µg) were performed and the samples were electrophoresed through a 0.8% agarose gel and blotted onto nylon (Hybond N +, Amersham) filters. Prehybridization and hybridization procedure were performed as described above and final washes were at 45°C in 0.1 X SSC, 0.05% SDS.

For Northern blot analysis, total RNA extracted from mouse or cultured cells was denatured in Glyoxal-DMSO, electrophoresed through a 1% agarose gel and transferred onto a nylon membrane (Hybond N +, Amersham). Prehybridization and hybridization, as well as washing were performed as described above, except that hybridization was performed in the presence of 10% dextran sulphate.

### EXAMPLE 3

### In situ hybridization

For chromosal localization of the β3-adrenergic receptor gene in mouse, in situ hybridization was performed on metaphase spreads of concavalin A-stimulated lymphocytes from a WMP male mouse, in which all the autosomes except number 19 were in the form of metacentric Robertsonian translocations. For localization of the β3-adrenergic receptor gene in man, metaphase spreads were from phytohaemagglutinin-stimulated human lymphocytes. In both cases the lymphocytes were cultured at 37°C for 72 hrs., with 5-bromodeoxyuridine (60 µg/ml) added for the final 7 hours of culture.

Specific probes were the 310 bp A43 fragment of the mouse β3-adrenergic receptor gene and a 206 bp (AccI-ApaLI) DNA fragment encompassing the third intracytoplasmic loop of the human β3-adrenergic receptor. These fragments were subcloned into the pUC19 plasmid vector, tritium labelled by nick translation to a specific activity of 10⁸ d.p.m./µg, and used at a final concentration of 25 ng/ml of hybridization solution. Hybridization to metaphase spreads, post-hybridization washes, emulsion autoradiography, R-banding and silver grain analysis were carried out as described by Mattei et al. HUMAN GENETICS, 69, pgs. 268-271 (1985).

### EXAMPLE 4

### Construction, cell culture and transfections

For expression in eukaryotic cells, a 1365 bp NarI-BamHI restriction fragment from the mouse β3-adrenergic receptor gene was inserted under the control of the SV40 early promoter into a plasmid vector containing the 3'-untranslated region of the gene for the hepatitis-B surface antigen, and murine dihydrofolate reductase (DHFR) gene as a selectable marker as described by Larsky et al. Biotechnology, 2, pgs. 527-530 (1984). The resulting construct contained 15 bp of 5' untranslated region, 1164 bp of coding region and 186 bp of 3' non-coding region of the mouse β3-adrenergic receptor gene.

CHO cells, deficient in DHFR, were grown in Ham's F12 medium (Seromed) supplemented with 10% fetal calf serum, 2 mM L-glutamine, 15 µg/ml glycine, 9.5 µg/ml hypoxanthine and 1.46 µg/ml thymidine. Transfection with 5 µg of the expression vector was performed by the calcium phosphate precipitation method as described by Tate et al. EUR. J. Biochem., 196, pgs. 357-361 (1991). Stable transformants expressing the DHFR gene were screened for the expression of β adrenergic receptor by stimulation of cAMP accumulation with 1 µM (-)-isoproterenol. One clone giving the highest stimulation was subcloned by limiting dilution, and is referred to as CHO-Moβ3'.

### EXAMPLE 5

### Isolating and sequencing of the human β3AR gene.

Similar procedures set forth in the above examples to isolate and sequences the murine β3-adrenergic receptor gene were used in the isolation of the human β3-adrenergic receptor gene. A human genomic library was screened with the entire coding regions of the gene for the β1-adrenergic receptor of turkey Yarden et al., P.N.A.S., 83, 6795 (1986) and of the human gene for the β2-adrenergic receptor Emorine et al P.N.A.S. 84, 6995 (1987). The library was constructed in the BamHI sites of the EMBL4 vector from size-selected (15 to 20 kb) fragments of human placental DNA partially digested with Sau3a. For plague purification, probes of a 1.3 kb NcoI-AhaI restriction fragment of the human β2-adrenergic receptor and the 1.8 kb fragment of the turkey β1-adrenergic receptor were used Yarden et al., P.N.A.S., 83, 6795 (1986).

Among 43 positive clones two carried the gene coding for the human β1-adrenergic receptor and another two the gene for the β2-adrenergic receptor.

A family of 14 homologous clones consisting of a group of six identical clones, a second group of three identical clones and a third group of two identical clones and three independent clones displayed sequences homologous to both probes in a 2.1 kb BamHI and BglII fragment. From one clone, this 2.1 kb fragment was sequenced using the methods described by the chain termination method of Sanger et al., P.N.A.S., 74, 5463 (1977). This fragment that was entirely sequenced and shown to contain a gene coding for a polypeptide of 402 amino acids with a predicted size of 42,881 daltons.

### EXAMPLE 6

### Nucleotide sequence determination

2.5 kb SphI-BglII, and 2.2 kb XhoI-BglII fragments spanning the entire 3'-untranslated regions of the human and mouse β3-AR genomic genes, respectively, were inserted in both orientations into M13 and individual subclones were sequenced by the standard termination method (Taquence DNA sequencing kit, USB, Ohio). Primers were oliogonucleotides synthesized step by step during the course of sequence determination.

### EXAMPLE 7

### PCR-generated cDNA

Total RNA was prepared from frozen-powdered tissues and digested for 1 hr at 37°C with 0.3U of RNase-free DNase I (Promega, Wisconsin) per µg of nucleic acid in 100 mM Tris-HCl, pH 7.5; 50 mM MgCl₂, in the presence of 2 U/ml of placenta RNase inhibitor. 0.25 µg of RNA was then treated with 400 U of maloney murine leukemia virus reverse transcriptase (Gibco BRL) in 20 µl of PCR buffer containing 67 mM Tris-HCl, pH 8.4; 6.7 mM MgCl₂; 6.7 mM EDTA; 10 mM β-2-mercaptoethanol; 16 mM (NH₄)₂SO₄; and 0.1 mg/ml gelatine containing 0.4 mM each of dNTP, 10 mM random hexanucleotides and 2 U/ml RNase inhibitor. A control without reverse transcriptase was also run to ensure that amplification of the nucleotide sequence did not proceed from residual genomic DNA. 80 µl of PCR buffer containing 2.5 U of Thermophylus aquaticus polymerase (Perkin-Elmer-Cetus), 125 nM each of sense and antisense oligonucleotide primers, containing the following sequences, respectively: 5' GCTCCGTGGCCTCACGAGAA 3' and 5' CCCAACGGCCAGTGGCCAGTCAGCG 3', 125 mM each dNTP and 10% (v/v) dimethylsulfoxide was added to the cDNA samples which were submitted to 29 cycles at temperatures of 92°C, 1 minute; 57°C, 1.5 minutes; 72°C; 1.5 minutes; followed by 7 minutes of extension at 72°C in a temperature cycler (LEP-PREM).

### EXAMPLE 8

### Determination of mRNA transcription start sites

For nuclease S1 mapping single stranded DNA from a M13 subclone of the murine β3-AR gene promoter region (fragment BglII-NarI, was used as template for probe synthesis. After polymerization in the presence of α[³²P]-dATP, DNA was digested with Alw NI and the single stranded probe (380 nucleotides including 310 base from the β3-AR gene plus 70 bases from M13) was isolated on denaturing 6% polyacrylamide gels containing 7 M urea. Probe (10⁵ c.p.m.) and total RNA (50 µg) were mixed in 30 µl of 40 mM Pipes buffer pH 6.4, containing 0.4 M NaCl, 1 mM EDTA and 70% formamide and denatured for 10 min at 85°. After hybridization (14-16 hr at 30°C) Samples were digested for 30 min at 37°C with 100-200 units of S1 nuclease in 400 µl of 50 mM sodium acetate, pH 4.8, 280 mM NaCl, 4.5 mM ZnSO₄. Following incubation for 10 min at 65°C, samples were phenol-extracted, concentrated by ethanol precipitation and analyzed on 6% acrylamide, 7 M urea sequencing gels.

For primer extension, oligonucleotide A74 (36-mer) having the following sequence: 5' CTGGTCCAGGGGAGGGGACAGCAAGGCATGAGAGCG 3' was labeled with T4-polynucleotide kinase hybridized (5 x 10⁵ c.p.m.) to 50 µg of total RNA, in 30 µl of 20 mM Tris-HCl, pH 8, 50 mM NaCl, 10 mM MgCl₂, 1 mM dithiotreitiol, and 0.1 mM EDTA, for 14-16 hr at 37°C. Nucleic acids were ethanol precipitated and incubated for 90 min at 42°C after resuspension in 25 µl of 50 mM Tris-HCl, pH 8.3, 8 mM MgCl₂, 30 mM KCl containing 0.5 mM each dNTP, 50 units of placenta RNase inhibitor, and 40 units of avian myeloblastosis virus reverse transcriptase. Reactions were terminated by addition of 1 µl of both 0.5 M EDTA and pancreatic RNase A (1 mg/ml) and incubation for 30 min at 37°C was performed. After phenol-extraction and ethanol precipitation in the presence of ammonium acetate (2 M final) samples were analyzed on 6% acrylamide, 7 M urea sequencing gels.

### EXAMPLE 9

### Expression of the entire human β3-adrenergic receptor

For expression in eukaryotic cells, a 3.7 kb SmaI-BglII restriction fragment from the human β3-adrenergic receptor gene was inserted under the control of the SV40 early promoter into a plasmid vector also containing the murine dihydrofolate reductase (DHFR) gene as a selectable marker as described by Larsky et al. Biotechnology, 2, pgs. 527-530 (1984). The resulting construct contained 4 bp of 5' untranslated region, the entire intron/exon sequence of the human β3-receptor gene with its own 3' untranslated region.

CHO cells or L cells (wherein the splicing of the gene can take place) deficient in DHFR, were grown in Ham's F12 medium (Seromed) supplemented with 10% fetal calf serum, 2 mM L-glutamine, 15 µg/ml glycine, 9.5 µg/ml hypoxanthine and 1.46 µg/ml thymidine. Transfection with 5 µg of the expression vector was performed by the calcium phosphate precipitation method as described by Tate et al. EUR. J. Biochem., 196, pgs. 357-361 (1991). Stable transformants expressing the DHFR gene were screened for the expression of β adrenergic receptor by stimulation of cAMP accumulation with 1 µM (-)-isoproterenol.

### EXAMPLE 10

### Testing of various chemicals for β3-adrenergic receptor affinity

Preconfluent cells (5 x 10⁵) were incubated at 37°C for 20 minutes in 0.5 ml of Hank's buffer containing 20 mM HEPES (pH 7.4), 1 mM ascorbic acid, 1 mM isobutylmethylxanthine and various concentrations of agonists such as BRL 37344, (-)-isoproterenol and (-)-norepinephrine. After boiling for 5 minutes and centrifuging at 4,000 r.p.m. for 10 minutes at 4°C, the amount of cAMP produced was measured using the Amersham cAMP assay kit. Inhibition studies for cAMP accumulation were carried out by preincubating cells at 37°C for 10 minutes with an antagonist such as propanolol, ICI 118551 and CGP 20712A before the addition of 5 nM (-)-isoproterenol. The mixture was incubated for an additional 20 minutes. Computer analysis of the data was carried out using the Graph-PAD program (copyright 1987 by M.J. Motulsky).

## Claims

1. An isolated and purified nucleic acid sequence comprising the nucleotide sequence of Figure 1 encoding a β3-adrenergic receptor.

2. An isolated and purified nucleic acid sequence comprising the nucleotide sequence of Figure 2 encoding a β3-adrenergic receptor.

3. An isolated and purified nucleic acid sequence containing the intron sequences set forth in Figures 1 or 2.

4. A recombinant vector for cloning or expression, said vector selected from the group of a plasmid, cosmid and phage comprising the nucleotide sequence according to Claim 1 or Claim 2.

5. A cell host transformed by a recombinant vector according to Claim 4 which comprises the elements of regulation allowing the expression of the nucleotide sequence according to Claim 1 or Claim 2.

6. A transformed host cell according to Claim 5, wherein said host cell is an eukaryotic cell.

7. The transformed host cell according to Claim 6, wherein said eukaryotic cell is a CHO cell or a L cell.

8. A nucleotide probe containing the nucleotide sequence of Figures 1 or 2 wherein said nucleotide probe is capable of hybridizing with the gene encoding a β3- adrenergic receptor.

9. The nucleotide probe of Claim 8, wherein said nucleotide probe is capable of hybridizing with a complementary sequence under conditions of hybridization such that it does not hybridize with the genes or messenger RNA of β1 or β2 adrenergic receptors.

10. A process for the preparation of a β-3 adrenergic receptor comprising the steps of:
(a) transforming a host cell with a recombinant vector containing the nucleic sequences of Claims 1 or 2;
(b) culturing said transformed host cell in a culture medium; and
(c ) recovering said polypeptide produced by the cells.

11. A nucleotide probe of claims 8 and 9, wherein said probe is used to detect functional abnormalities in the β-3 adrenergic receptor gene.

12. A nucleotide probe of claims 8 and 9, wherein said probe is used to detect functional abnormalities in the β-3 adrenergic receptor sequence.

## Patentansprüche

1. Isolierte und gereinigte Nukleinsäuresequenz, umfassend die Nukleotidsequenz von Figur 1, die einen β3-adrenergen Rezeptor codiert.

2. Isolierte und gereinigte Nukleinsäuresequenz, umfassend die Nukleotidsequenz von Figur 2, die einen β3-adrenergen Rezeptor codiert.

3. Isolierte und gereinigte Nukleinsäuresequenz, die die in den Figuren 1 oder 2 aufgeführten Intronsequenzen enthält.

4. Rekombinanter Vektor für Klonierung oder Expression, wobei der Vektor aus der Gruppe eines Plasmids, Cosmids und Phagens ausgewählt ist und die Nukleotidsequenz nach Anspruch 1 oder Anspruch 2 umfasst.

5. Wirtszelle, die durch einen rekombinanten Vektor nach Anspruch 4 transformiert ist und die Regulationselemente umfasst, die die Expression der Nukleotidsequenz nach Anspruch 1 oder Anspruch 2 erlauben.

6. Transformierte Wirtszelle nach Anspruch 5, wobei die Wirtszelle eine eukaryotische Zelle ist.

7. Transformierte Wirtszelle nach Anspruch 6, wobei die eukaryotische Zelle eine CHO-Zelle oder eine L-Zelle ist.

8. Nukleotidsonde, die die Nukleotidsequenz von Figur 1 oder 2 enthält, wobei die Nukleotidsonde in der Lage ist, mit dem einen β3-adrenergen Rezeptor codierenden Gen zu hybridisieren.

9. Nukleotidsonde nach Anspruch 8, wobei die Nukleotidsonde in der Lage ist, mit einer komplementären Sequenz zu hybridisieren unter solchen Hybridisierungsbedingungen, dass sie mit den Genen oder der mRNA von β1- oder β2-adrenergen Rezeptoren nicht hybridisiert.

10. Verfahren zur Herstellung eines β3-adrenergen Rezeptors, das die Schritte umfasst:
(a) eine Wirtszelle mit einem rekombinanten Vektor, der die Nukleinsequenzen nach Anspruch 1 oder 2 enthält, zu transformieren;
(b) die transformierte Wirtszelle in einem Kulturmedium zu züchten und
(c) das durch die Zellen produzierte Polypeptid zu gewinnen.

11. Nukleotidsonde nach den Ansprüchen 8 und 9, wobei die Sonde verwendet wird, um funktionelle Abnormalitäten in dem Gen des β3-adrenergen Rezeptors zu detektieren.

12. Nukleotidsonde nach den Ansprüchen 8 und 9, wobei die Sonde verwendet wird, um funktionelle Abnormalitäten in der Sequenz des β3-adrenergen Rezeptors zu detektieren.

## Revendications

1. Séquence d'acides nucléiques isolée et purifié comprenant la séquence de nucléotides de la figure 1 codant pour un récepteur β3-adrénergique.

2. Séquence d'acides nucléiques isolée et purifiée comprenant la séquence de nucléotides de la figure 2 codant pour un récepteur β3-adrénergique.

3. Séquence d'acides nucléiques isolée et purifiée contenant les séquences intron exposées dans les figures 1 ou 2.

4. Vecteur recombinant pour clonage ou expression, ledit vecteur est choisi dans le groupe formé d'un plasmide, d'un cosmide et d'un phage comprenant la séquence de nucléotides selon la revendication 1 ou la revendication 2.

5. Hôte cellulaire transformé par un vecteur recombinant selon la revendication 4 qui comprend les éléments de régulation permettant l'expression de la séquence de nucléotides selon la revendication 1 ou la revendication 2.

6. Cellule hôte transformée selon la revendication 5, dans laquelle ladite cellule hôte est une cellule eucaryote.

7. Cellule hôte transformée selon la revendication 6, dans laquelle ladite cellule eucaryote est une cellule CHO ou une cellule L.

8. Sonde de nucléotides contenant la séquence de nucléotides des figures 1 ou 2 dans laquelle ladite sonde de nucléotides est capable de s'hybrider avec le gène codant pour un récepteur β3-adrénergique.

9. Sonde de nucléotides selon la revendication 8, dans laquelle ladite sonde de nucléotides est capable de s'hybrider avec une séquence complémentaire dans des conditions d'hybridation telles qu'elle ne s'hybride pas avec les gènes ou l'ARN messager des récepteurs β1- ou β2-adrénergiques.

10. Procédé pour la préparation d'un récepteur β3-adrénergique comprenant les étapes consistant :
(a) à transformer une cellule hôte avec un vecteur recombinant contenant les séquences d'acides nucléiques des revendications 1 ou 2 ;
(b) à cultiver ladite cellule hôte transformée dans un milieu de culture ; et
(c) à récupérer ledit polypeptide produit par les cellules.

11. Sonde de nucléotides selon les revendications 8 et 9, dans laquelle ladite sonde est utilisée pour détecter des anomalies fonctionnelles dans le gène du récepteur β3-adrénergique.

12. Sonde de nucléotides selon les revendications 8 et 9, dans laquelle ladite sonde est utilisée pour détecter des anomalies fonctionnelles dans la séquence du récepteur β3-adrénergique.
